# EUROPEAN PATENT SPECIFICATION

(11) **EP 3 522 951 B1**
(45) Date of publication and mention of the grant of the patent: **02.12.2026**
(21) Application number: 17787733.9
(22) Date of filing: 04.10.2017
(51) Int. Cl.: A61M 5/19, A61M 5/28

(54) **MULTI-STAGE DELIVERY SYSTEM**
MEHRSTUFIGES ABGABESYSTEM
SYSTÈME DE DISTRIBUTION MULTI-ÉTAPES

(30) Priority: 07.10.2016 US 201662405489 P
(43) Date of publication of application: 14.08.2019
(73) Proprietor: Insulet Corporation, Acton, MA 01720 (US)
(72) Inventor: NAZZARO, David, Groveland, MA 01834 (US)
(74) Representative: Peterreins Schley
(86) International application number: PCT/US2017/055054
(87) International publication number: WO 2018/067645

(56) References cited:
- EP-A1- 2 468 338
- WO-A1-2014/149357
- WO-A1-2015/117854
- JP-A- H08 238 324
- US-A- 4 755 169
- US-A1- 2003 109 827
- US-A1- 2008 114 304

## Description

### TECHNICAL FIELD

Embodiments generally relate to medication delivery. More particularly, embodiments relate to drug delivery systems for delivering multiple different drugs.

### BACKGROUND

Many patients may be required to receive dosages of different drugs. Conventional drug delivery systems, such as many conventional wearable drug delivery devices, typically only provide a dosage of a single drug. Therefore, a patient may be required to use multiple conventional drug delivery devices to receive the different drug dosages. With each additional device, the patient is required to insert a new needle to facilitate delivery of each drug. Accordingly, what is needed is a drug delivery system that can deliver multiple different drugs and dosages in a less burdensome manner while reducing patient discomfort.

US 2003/109827 A1 discloses a system and method for delivering drug solutions to a user, the system having a housing with a drug reservoir therein. The system also includes an activation assembly having a delivery needle and an activation device for activating delivery. The system further includes a pump, associated with the drug reservoir and a flow restrictor. When activated, the pump moves the drug out of the drug reservoir through the delivery needle and into the skin of the user. The pump may be in the form of a spring.

### BRIEF DESCRIPTION OF THE DRAWINGS

FIG. 1 illustrates an exemplary multiple stage (multi-stage) drug delivery system.
FIG. 2 illustrates the multi-stage drug delivery system of FIG. 1 in a first stage of operation.
FIG. 3 illustrates the multi-stage drug delivery system of FIG. 1 in a second stage of operation.
FIG. 4 illustrates the multi-stage drug delivery system of FIG. 1 in a third stage of operation.
FIG. 5 illustrates the multi-stage drug delivery system of FIG. 1 in a fourth stage of operation.
FIG. 6 illustrates the multi-stage drug delivery system of FIG. 1 in a fifth stage of operation.
FIG. 7 illustrates the multi-stage drug delivery system of FIG. 1 in a sixth stage of operation.
FIG. 8 illustrates an exemplary method for delivering multiple liquid drugs to a patient.

### SUMMARY

The present invention is defined in the appended claims.

### DETAILED DESCRIPTION

This disclosure presents various systems, components, and methods related to a drug delivery system. Each of the systems, components, and methods disclosed herein provides one or more advantages over conventional systems, components, and methods.

Various examples provide a multiple stage (multi-stage) drug delivery system and method of use. The multi-stage drug delivery system can include two or more chambers for storing liquid drugs. The multi-stage drug delivery system can deliver each drug to a user in succession. The multi-stage drug delivery system can include the same drugs or different drugs. The multi-stage drug delivery system can store and dispense the same amount of each drug or different amounts of each drug. Each drug can be delivered at a desired rate over a desired amount of time. The multi-stage drug delivery system can interface with a variety of different fluid delivery mechanisms to pass the stored drugs to the user. The multi-stage drug delivery system provides a simplified architecture for storing and dispending multiple different drugs to the user within the same container, enabling the user to use a same needle insertion to deliver the drugs. The multi-stage drug delivery device can be implemented within an on-body or wearable drug delivery device. Other embodiments are disclosed and described.

Various embodiments include a multi-stage drug delivery system having a cartridge, a first plunger positioned in the cartridge, and a second plunger positioned in the cartridge with the second plunger spaced apart from the first cartridge. The first plunger can be accessible through a first end of the cartridge. The first plunger, the second plunger, and the cartridge can form a first chamber configured to store a first liquid drug. The second plunger and the cartridge can form a second chamber configured to store a second liquid drug. A cannula can pierce the first plunger to access the first liquid drug. As the first plunger is driven toward the second plunger, the first liquid drug can be expelled from the first chamber for delivery to a patient through the cannula. After expelling the first liquid drug, the cannula can pierce the second plunger to access the second liquid drug. As the first and second plungers are together driven toward a closed end of the cartridge, the second liquid drug can be expelled from the second chamber for delivery to the patient through the cannula. The multi-stage drug delivery system allows two or more drugs to be delivered to a patient at different predetermined times, in different amounts, and according to different rates.

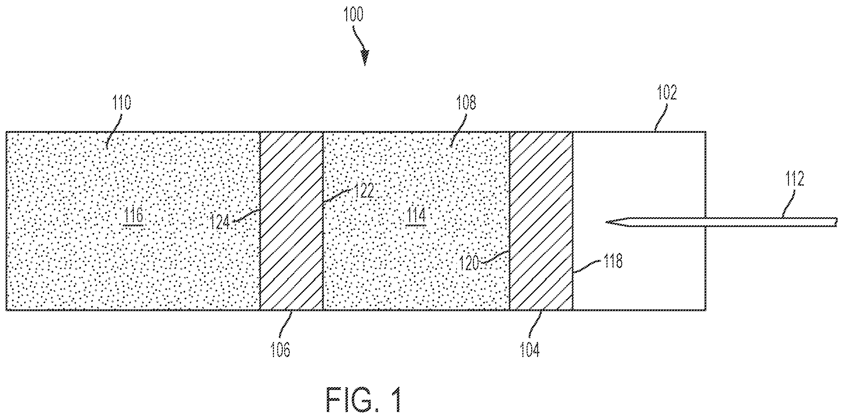

FIG. 1 illustrates a multi-stage drug delivery system 100. The multi-stage drug delivery system 100 can include a cartridge 102, a first plunger 104, and a second plunger 106. The multi-stage drug delivery system 100 can further include a first drug 108 and a second drug 110. The first and second drugs 108 and 110 can be any therapeutic agent including liquid drugs. The first and second drugs 108 and 110 can be different therapeutic agents or drugs. The multi-stage drug delivery system 100 can separately store and dispense the first and second drugs 108 and 110 using a common or shared drug delivery mechanism while enabling each drug to be dispensed at different times according to different customized schedules (e.g., different rates of delivery). The cartridge 102 can be of any size or shape. As an example, the cartridge 102 can be cylindrical with a circular cross-sectional shape.

As shown in FIG. 1, the first drug 108 can be stored in a first chamber 114 defined by the first plunger 104, the second plunger 106, and the cartridge 102. The storage and use of the first drug 108 in the first chamber 114 can represent a first stage of the multi-stage drug delivery system 100. The second drug 110 can be stored in a second chamber 116 defined by the second plunger 106 and the cartridge 102. The storage and use of the second drug 110 in the second chamber 116 can represent a second stage of the multi-stage drug delivery system 100.

The first and second drugs 108 and 110 can be hermetically separated and sealed by the cartridge 102 and the first and second plungers 104 and 106 as shown in FIG. 1. The first and second plungers 104 and 106 can be elastomeric plungers. The first and second drugs 108 and 110 can be provided in a common device (e.g., the cartridge 102) while ensuring the first and second drugs 108 and 110 are separately stored and dispensed. The first and second plungers 104 and 106 can be positioned anywhere along an interior portion of the cartridge 102.

The multi-stage drug delivery system 100 can be used in a drug delivery device that provides the first and second drugs 108 and 110 to a user. For example, the multi-stage drug delivery system 100 can be part of a bolus device and/or a wearable drug delivery device. The multi-stage drug delivery system 100 can include the same or different amounts of the first and second drugs 108 and 110 (e.g., the dosages of the first and second drugs 108 and 110 can be the same or different). The first drug 108 can be dispensed first over a first amount of time at a first rate of delivery with the second drug 110 dispensed over a second amount of time at a second rate of delivery, after the first drug 108 is dispensed. The multi-stage drug delivery system 100 enables the first and second drugs 108 and 110 to be provided to the user using the same fluid path to the user. Consequently, a single needle insertion can be used to deliver the first and second drugs 108 and 110.

The multi-stage drug delivery system 100 can dispense the second drug 110 immediately after the first drug 108 has been dispensed or can deliver the second drug 110 after a delay. Accordingly, the first and second drugs 108 and 110 can be delivered relatively close together in time or can be delivered at separate times after a desired delay. Further, the delivery of the first and second drugs 108 and 110 can be customized in terms of the rate at which each drug is delivered. For example, the first drug 108 can be delivered over a first amount of time while the second drug can be delivered over a second, different amount of time. The first and second amounts of time can be varied as desired to dispense the first and second drugs 108 and 110 at different desired rates of delivery, respectively. In this way, a customized delivery of the first and second drugs 108 and 110 can be provided with the dosages (e.g., how much drug to dispense in total), delivery times (e.g., when to dispense a drug), and delivery schedules (e.g., how quickly or slowly to dispense the dosage) for each drug being largely independent of each another.

FIG. 1 shows the multi-stage drug delivery system 100 in an initial state. Specifically, no mechanical energy or force is introduced or applied to the multi-stage drug delivery system 100 as shown in FIG. 1. A cannula 112 is shown in relatively close proximity to the multi-stage drug delivery system 100. The cannula 112 can be a hard cannula 112 that is sterilized. The cannula 112 can be positioned adjacent to the cartridge 102 or within an open area of the cartridge 102 yet still displaced from the first plunger 104. The cannula 112 can initially be positioned entirely outside of the cartridge 102. FIG. 1 can represent the multi-stage drug delivery system 100 before activation (e.g., before activation of a wearable drug delivery device in which the multi-stage drug delivery system 100 operates).

As shown in FIG. 1, the cannula 112 can be positioned adjacent to a first end of the cartridge 102. In particular, the cannula 112 is positioned in proximity to a first surface 118 of the first plunger 104. The first surface 118 can be adjacent to an open end of the cartridge 102 which allows the cannula 112 to enter the cartridge 102. The first plunger 104 can further include a second surface 120. The second surface 120 can be adjacent to the first drug 108. The second plunger 106 can also include a first surface 122 and a second surface 124. The first surface 122 can be adjacent to the first drug 108 and the second surface 124 can be adjacent to the second drug 110 as shown.

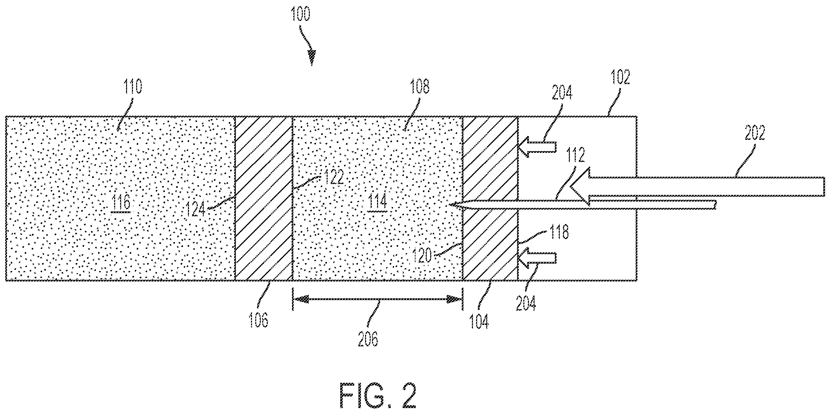

FIG. 2 illustrates insertion of the cannula 112 into the first stage of the multi-stage drug delivery system 100 (e.g., into the first chamber 114). As shown in FIG. 2, indicator 202 indicates application of a force applied to the cannula 112. The application of the force 202 to the cannula 112 can cause the cannula 112 to pierce and pass through the first plunger 104. The force 202 can be such that the cannula 112 pierces through the first plunger 104 to enter the first stage (e.g., the first chamber 114) holding the first drug 108. The force 202 can cause the cannula to enter the first chamber 114 at a desired depth. For example, the cannula 112 can pierce the first plunger 104 and enter the first stage at a depth so as to maximize extraction of the first drug 108 stored in the first chamber 114. In various embodiments, the body of the first plunger 104 may be counter-bored to facilitate piercing by the cannula 112.

As shown in FIG. 2, to enter the first chamber 114 holding the first drug 108, the cannula 112 can be caused to pierce the first surface 118 and the second surface 120 of the first plunger 104 and to extend through the first plunger 104. A tip or end or the cannula 112 can be sharp or pointed to facilitate piercing of the first plunger 104. To obtain access to the first chamber 114, the cannula 112 can be caused to move in a lateral direction toward a far end of the cartridge 102 as shown. The tip or end of the cannula 112 can be positioned near to the second surface 120 of the first plunger 104 once access to the first chamber 114 is provided. The cannula 112 can be positioned at a center of the first plunger 104 but is not so limited. In various embodiments, the first plunger 114 can be inserted along any portion of the first surface 118 of the first plunger 104 for example.

Once the cannula 112 is inserted into the first stage of the multi-stage drug delivery system 100 at a desired depth, the first drug 108 can be introduced into the fluid delivery mechanism coupled to the cannula 112. As an example, the cannula 112 can be coupled to a fluid delivery mechanism that delivers the first drug 108 to a user or patient. A variety of fluid delivery mechanisms can be used. For example, the fluid delivery mechanism can include one or more components for coupling the cannula 112 to the patient. In various embodiments, the components can include tubing (e.g., plastic and/or stainless steel tubing) coupled to the cannula as well as a needle or cannula (e.g., coupled to the tubing) for accessing a site on the patient. Overall, the fluid delivery mechanism can be or can provide a fluid path from the cannula to the user of the multi-stage drug delivery system 100. Accordingly, when the cannula 112 is coupled to a liquid drug, then the fluid path can be used to deliver the liquid drug to the user. In various embodiments, the cannula 112 can be part of the delivery mechanism or can be coupled to it. In various embodiments, the cannula 112 can be coupled to the patient.

The fluid delivery mechanism can further be used to apply the force 202. As an example, the fluid delivery mechanism can include a cannula insertion component for driving the cannula 112 in a direction indicated by the applied force 202 to pierce the first plunger 104. The cannula insertion component can be coupled to the cannula 112 and can be triggered to cause the cannula 112 to advance toward the first plunger 104 to pierce the first plunger 104. In various embodiments, the cannula insertion component can comprise a mechanical system or an electromechanical system for manipulating the cannula 112. In various embodiments, the cannula insertion component can include one or more springs such as, for example, an expansion spring, a compression spring, and/or a torsion spring. The cannula insertion component can be trigger to pierce through the first plunger 104 based on a user input or action that indicates a desire to activate delivery of the first drug 108 (e.g., by a user pressing a button on a wearable drug delivery device to activate the device).

Further, the fluid delivery mechanism can include a drive component to apply a force 204 on the first plunger 104 to force the first drug 108 out of the first stage of the multi-stage drug delivery system 100. For example, after the cannula 112 pierces the first plunger 104 and is positioned at a desired depth within the first chamber 114, a second force can be applied to the first plunger 104 as indicated by indicators 204. The force 204 can be applied to any portion of the first surface 118 of the first plunger 104 (e.g., at one or more positions along the first surface 118). The force 204 can drive the first drug 108 out of the first chamber 114, through the fluid delivery mechanism (e.g., the cannula 112 and any other components coupling the cannula to the patient), and on to the user. The force 204 can cause the first plunger 104 to move towards the stationary second plunger 106. The drive component can be coupled to the first plunger 104. In various embodiments, the drive component can comprise a mechanical system or an electromechanical system for driving the first plunger 104 toward the far end of the cartridge 102. In various embodiments, the drive component can include one or more springs such as, for example, an expansion spring, a compression spring, and/or a torsion spring.

The applied force 204 can be constant or varied and can be used to deliver the first drug 108 to the user over a desired amount of time (e.g., at a desired rate of delivery). For example, a relatively strong force 204 can be applied to rapidly provide the first drug 108 to the user. Alternatively, a relatively weak force 204 can be applied to slowly provide the first drug 108 to the user. In general, any delivery schedule and rate of delivery of the first drug 108 can be provided by using and varying the force 204.

FIG. 2 also shows a stroke 206 of the multi-stage drug delivery system 100 - that is, the distance the first plunger 104 travels to expel all or approximately all of the first drug 108 from the first chamber 114 of the multi-stage drug delivery system 100 (e.g., the stroke 206 can represent the amount of the first drug 108 that can be stored and provided to a user). As discussed above, the stroke 206 can be varied by the amount of the first drug 108 included in the multi-stage drug delivery system 100 and the magnitude and timing of the force 204 applied to the first plunger 104 can determine how the amount of the first drug 108 is provided to the user. FIG. 2 can represent a state of the multi-stage drug delivery system 100 when the first drug 108 is initially accessed (e.g., when the cannula 112 is first introduced into the first chamber 114). FIG. 2 can represent the multi-stage drug delivery system 100 after activation and as delivery of the first drug 108 to the user first begins.

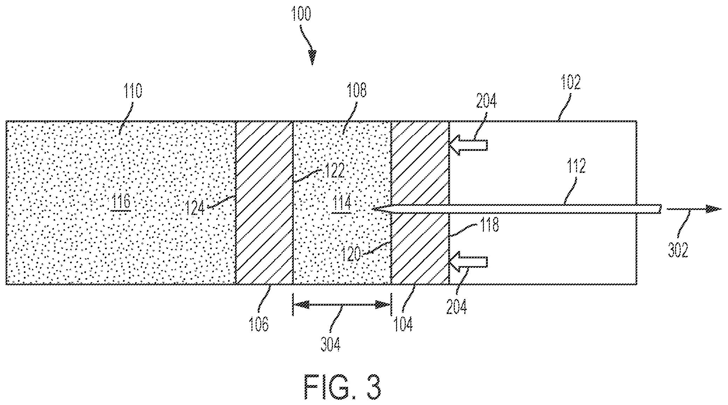

FIG. 3 illustrates extraction of the first drug 108 from the multi-stage drug delivery system 100. Specifically, FIG. 3 shows the first plunger 104 being driven towards the second plunger 106. The second plunger 106 can be in a fixed position. As a result, the first drug 108 can be forced out of the first chamber 114 of the multi-stage drug delivery system 100, through the cannula 112, and on to the fluid delivery mechanism coupled to the cannula 112 for delivery to the user. As shown in FIG. 3, indicator 302 represents the first liquid drug 108 from the first chamber 114 being expelled out of the first chamber 114 through the cannula 112. The first liquid drug 108 can flow out of the cartridge 102 in a direction that is substantially opposite to the direction of the movement of the first plunger 104 towards the second plunger 106 which causes the first liquid drug 108 to be expelled out of the first chamber 114.

As shown in FIG. 3, the force 204 can be applied to the first plunger 104 to drive it towards the second plunger 106. The inserted position of the cannula 112 can remain in relatively the same position with respect to the first plunger 104 to ensure maximize extraction of the first drug 108. A reduction in stroke 304 is also shown for reference in FIG. 3. The reduction in stroke 304 can indicate that the size of the first chamber 114 of the multi-stage drug delivery system 100 has been reduced and that a corresponding amount of the first drug 108 has exited the multi-stage drug delivery system 100 through the cannula 112 for delivery to the user. FIG. 3 can represent a state of the multi-stage drug delivery system 100 when the first drug 108 is being extracted.

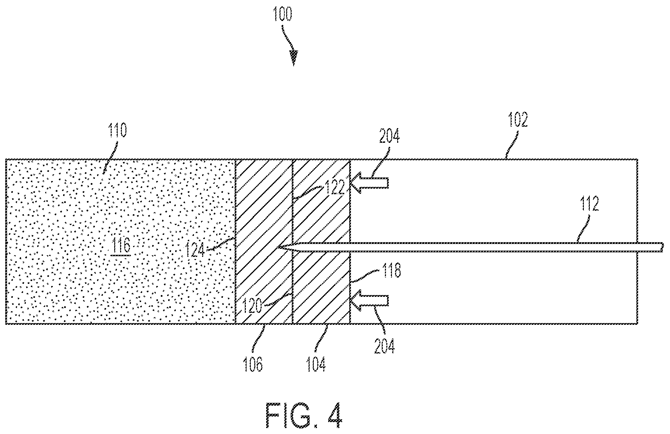

FIG. 4 illustrates the multi-stage drug delivery system 100 after depletion of the first drug 108. That is, FIG. 4 shows the multi-stage drug delivery system 100 after all or approximately all of the first drug 108 has been extracted from the first chamber 114 of the multi-stage drug delivery system 100. As shown in FIG. 4, the first plunger 104 is in close proximity to the second plunger 106 (e.g., adjacent such that no portion of the first drug 108 remains between the first and second plungers 104 and 106). The second surface 120 of the first plunger 104 can be adjacent to or pressed against the first surface 122 of the second plunger 106.

The force 204 applied to the first plunger 104 can drive the first plunger 104 to the position shown in FIG. 4. As the first drug 108 is being depleted and as the first plunger 104 is forced against the second plunger 106, the cannula 112 can be introduced into the second plunger 106. As shown in FIG. 4, a portion of the end of the cannula 112 can pierce a portion of the second plunger 106. The cannula 112 can pierce the first surface 122 of the second plunger 106 and can be partially inserted into the second plunger 106. The state of the multi-stage drug delivery system 100 as shown in FIG. 4 can be a state following depletion of the first drug 108 and prior to extraction of the second drug 110.

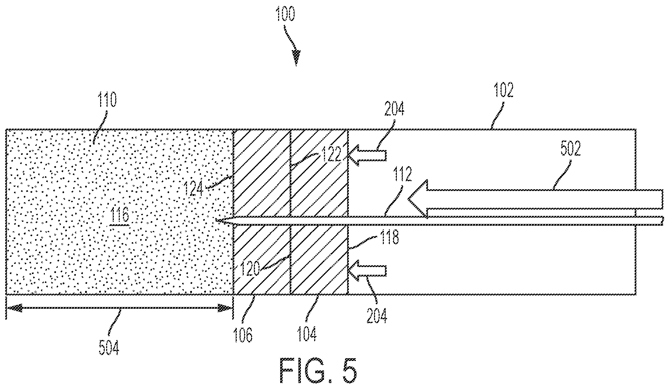

FIG. 5 illustrates insertion of the cannula 112 through the second plunger 106 of the multi-stage drug delivery system 100 and into the second chamber 116. As shown in FIG. 5, a second force 502 can be applied to the cannula 112 to drive the cannula 112 through the second plunger 106. The end of the cannula 112 can extend into the second chamber 116 of the multi-stage drug delivery system 100 a desired amount to ensure maximum extraction of the second drug 110. The cannula 112 can extend beyond the second surface 124 of the second plunger to be coupled to the second liquid drug 110 stored in the second chamber 116.

When the cannula 112 is inserted through the second plunger 106 and into the second chamber 116 of the multi-stage drug delivery system 100, the second drug 110 can be introduced into the fluid path coupled to the user (e.g., a fluid path coupled to the cannula 112). Specifically, the second drug 110 can be provided to the delivery mechanism coupled to the cannula 112 so that the second drug 110 may be passed on to the user. As shown, the force 204 can be applied to the first plunger 104 to move both the first plunger 104 and the second plunger 106 toward the end of the cartridge 102. That is, by applying the force 204 to the first plunger 104, the second plunger 106 is forced to move toward an end of the cartridge 102. In turn, the volume or size of the second chamber 116 is reduced which forces the second drug 110 through the cannula 112 and on to the fluid delivery mechanism.

FIG. 5 also shows a stroke 504 for the second chamber 116 of the multi-stage drug delivery system 100 - that is, the distance the second plunger 106 travels to expel all or approximately all of the second drug 110 from the second chamber 116 of the multi-stage drug delivery system 100 (e.g., the amount of the second drug 110 that can be stored and provided to a user). As discussed above, the stroke 504 can be varied by the amount of the second drug 110 included in the multi-stage drug delivery system 100 and the magnitude and timing of the force 204 applied to the first plunger 104 (and the second plunger 106) can determine how the amount of the second drug 110 is provided to the user. FIG. 5 can represent a state of the multi-stage drug delivery system 100 when the second drug 110 is initially accessed (e.g., when the cannula 112 is first introduced into the second chamber).

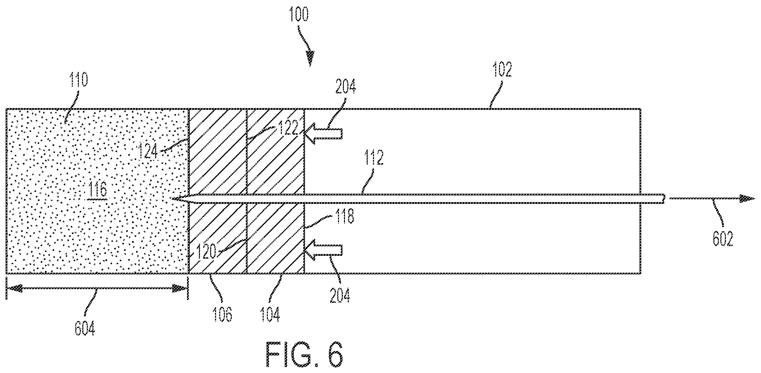

FIG. 6 illustrates extraction of the second drug 110 from the multi-stage drug delivery system 100. Specifically, FIG. 6 shows the first plunger 104 and the second plunger 106 together being driven toward the end of the cartridge 102. As a result, the second drug 110 can be forced out of the second chamber 116 of the multi-stage drug delivery system 100, through the cannula 112, and on to the fluid delivery mechanism coupled to the cannula 112 for delivery to the user. As shown in FIG. 6, the force 204 can be applied to the first plunger 104 (and to the second plunger 106 indirectly) to drive both the first and second plungers 104 and 106 towards the end of the cartridge 102. The inserted position of the cannula 112 can remain in relatively the same position with respect to the second plunger 106 to ensure maximize extraction of the second drug 110.

As shown in FIG. 6, indicator 602 represents the second liquid drug 110 from the second chamber 116 being expelled out of the second chamber 116 through the cannula 112. The second liquid drug 110 can flow out of the cartridge 102 in a direction that is substantially opposite to the direction of the movement of the first plunger 104 and the second plunger 106 towards the end of the cartridge 102 which causes the second liquid drug 110 to be expelled out of the second chamber 116.

A reduction in stroke 604 is also shown for reference in FIG. 6 - for example, to indicate the second chamber 116 of the multi-stage drug delivery system 100 has been reduced and that a corresponding amount of the second drug 110 has exited the multi-stage drug delivery system 100 for delivery to the user.

FIG. 6 illustrates the multi-stage drug delivery system 100 in a state of delivering the second drug 110 to a user. That is, FIG. 6 can represent a state after the cannula 112 has pierced through the second plunger 106 and the second drug 110 is being delivered to the user but prior to all of the second drug 110 being extracted.

In various embodiments, the force 502 can generally be applied to drive the cannula 112 through the plungers 104 and 106. The force 502 can then be removed or stopped and the force 204 can be applied or reapplied to drive the first plunger 104 (and the second plunger 106 when appropriate) towards the end (e.g., a closed end) of the cartridge 102. That is, the force 502 may not be used to drive the plungers 104 and 106 to the end of the cartridge 102. The force 204 can be applied along any portion of the first plunger 104 (e.g., including one or more positions along any portion of the first plunger 104).

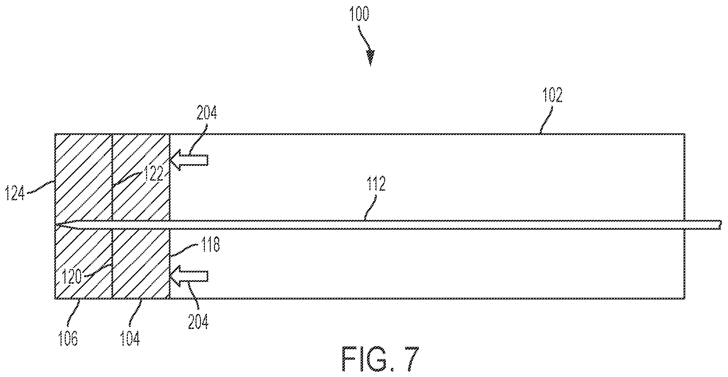

FIG. 7 illustrates the multi-stage drug delivery system 100 after depletion of the second drug 110. That is, FIG. 7 shows the multi-stage drug delivery system 100 after all or approximately all of the second drug 110 has been extracted from the second chamber 116 of the multi-stage drug delivery system 100. As shown in FIG. 7, the first and second plungers 104 and 106 are positioned against the end of the cartridge 102 (e.g., adjacent to the end of the cartridge 102 such that no portion of the second drug 108 remains between the second plunger 106 and the end of the cartridge 102).

As shown in FIG. 7, the second plunger 106 has met the end of stroke. Accordingly, the delivery of the dosage of the second drug 110 is substantially complete (e.g., all of the second drug 110 has been forced out of the second chamber 116). FIG. 7 can represent a state of the multi-stage drug delivery system 100 after both the first and second drugs 108 and 110 have been delivered to the user. The multi-stage drug delivery system 100 can be re-used (e.g., re-filled) or can be discarded.

FIGs. 1-7 illustrate the multi-stage drug delivery system 100 having two chambers (e.g., chambers 114 and 116) but is not so limited. That is, the multi-stage drug delivery system 100 can include any number of chambers (and corresponding number of plungers) to store and subsequently dispense any number of different drugs, in any amount, according to any desired delivery schedule in accordance with the techniques described herein. FIGs. 1-7 can represent cross-sectional side views of the multi-stage drug delivery system 100 during various stages of operation as described herein.

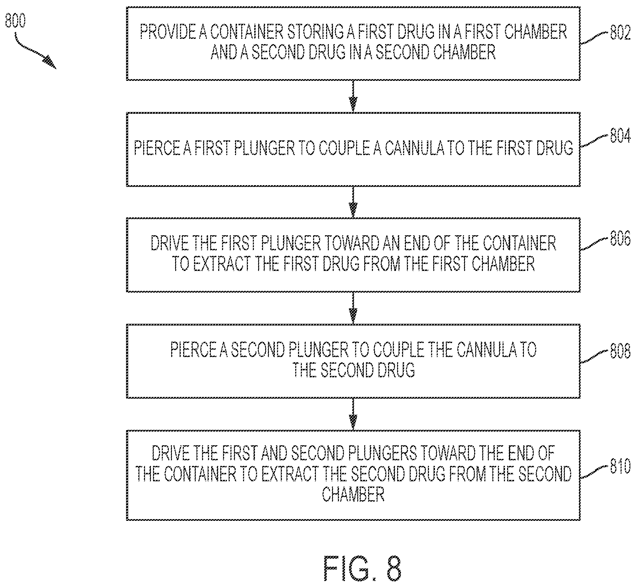

FIG. 8 illustrates a method 800 for delivering multiple liquid drugs to a patient using a multi-stage delivery system such as, for example, the multi-stage delivery system 100. The method 800 is described in relation to delivering two liquid drugs to the patient from the multi-stage delivery system but is not so limited. The method 800 is applicable to the delivery of any number of liquid drugs from a multi-stage delivery system configured to store a corresponding number of liquid drugs as will be appreciated by a person of ordinary skill in the art. Further, the method 800 is applicable to the delivery of different liquid drugs, to the delivery of different amounts of each liquid drug, and to the delivery of each liquid drug at different times and rates as will be appreciated by a person of ordinary skill in the art.

At 802, a container configured to store a first drug in a first chamber and configured to store a second drug in a second, different chamber can be provided. The first and second drugs can be liquid drugs. The first and second drugs can be any type of liquid drugs. The first and second liquid drugs can be the same or different drugs. The first and second chambers can be of the same size or can be configured to store different amounts of the first and second drugs, respectively.

The first and second drugs can be sealed and separated from each other. A first plunger positioned in the container, a second plunger positioned in the container and spaced apart from the first plunger, and the container can form the first chamber. The second plunger and the container can form the second chamber.

At 804, a cannula can pierce the first plunger. The cannula can be positioned at a center of the first plunger. The cannula can extend through the first plunger and into the first chamber. The cannula can extend into the first chamber by a desired amount or depth and can be coupled to the first drug.

The cannula can be coupled to the patient. Accordingly, when the cannula accesses the first drug stored in the first chamber the cannula can couple the first drug to the patient.

At 806, the first plunger is driven toward an end of the container. The first plunger is advanced further into the container toward the second plunger. The cannula can remain positioned in the first plunger as the first plunger is advanced. The second plunger can remain stationary. As a result of the first plunger moving further into the container, the first drug is expelled from the first chamber (e.g., as the size or volume of the first chamber is reduced). The expelled first drug can flow into and through the cannula and on to the patient.

The first plunger can be advanced to expel substantially all of the first drug. When substantially all of the first drug is expelled, the first plunger can be positioned against the second plunger.

At 808, the second plunger can be pierced by the cannula. The cannula can extend through the second plunger (and through the first plunger) and into the second chamber. The cannula can extend into the second chamber by a desired amount or depth and can be coupled to the second drug. Accordingly, when the cannula accesses the second drug stored in the second chamber the cannula can couple the second drug to the patient.

At 810, the first plunger and second plungers are driven toward the end of the container. The first and second plungers are advanced further into the container toward a closed end of the container. The cannula can remain positioned in the first and second plungers as the first and second plungers are advanced. As a result of the first and second plungers moving further into the container, the second drug is expelled from the second chamber (e.g., as the size or volume of the second chamber is reduced). The expelled second drug can flow into and through the cannula and on to the patient.

The first and second plungers can be advanced to expel substantially all of the second drug. When substantially all of the second drug is expelled, the second plunger can be positioned against the closed end of the container with the first plunger positioned against the second plunger.

Certain embodiments of the present invention were described above. It is, however, expressly noted that the present invention is not limited to those embodiments, but rather the intention is that additions and modifications to what was expressly described herein are also included within the scope of the invention. Moreover, it is to be understood that the features of the various embodiments described herein were not mutually exclusive and can exist in various combinations and permutations, even if such combinations or permutations were not made express herein, without departing from the scope of the invention. In fact, variations, modifications, and other implementations of what was described herein will occur to those of ordinary skill in the art without departing from the scope of the invention. As such, the invention is not to be defined only by the preceding illustrative description.

## Claims

1. A multiple stage drug delivery system, comprising:
a cartridge (102);
a first plunger (104) positioned in the cartridge (102);
a second plunger (106) positioned in the cartridge (102), wherein the first plunger (104), the second plunger (106), and the cartridge (102) form a first chamber (114) configured to store a first liquid drug (108), and the second plunger (106) and the cartridge (102) form a second chamber (116) configured to store a second liquid drug (110);
a cannula (112) configured to pierce through the first plunger (104) to access the first liquid drug (108) stored in the first chamber by application of a force (202) to the movable cannula (112); and
a cannula insertion component coupled to the cannula (112) and including one or more springs, the cannula insertion component configured to, in response to being triggered, cause the cannula (112) to pierce the first plunger (104), wherein:
the first plunger (104) is configured to:
move toward the second plunger (106), and
expel the first liquid drug (108) from the first chamber (114) through the cannula (112) in a direction that is substantially opposite to the direction of the movement of the first plunger (104) toward the second plunger (106), and
wherein the cannula (112) is further configured in response to a second force (502) applied to the cannula (112) to pierce the second plunger (106) to access the second liquid drug (110) stored in the second chamber (116) after the first liquid drug (108) is extracted from the first chamber (114).

2. The multiple stage drug delivery system of claim 1, further comprising a drive component configured to advance the first plunger (104) with the cannula toward the second plunger (106), thereby extracting the first liquid drug (108) from the first chamber (114) through the cannula (112).

3. The multiple stage drug delivery system of claim 2, wherein the drive component comprises one or more springs.

4. The multiple stage drug delivery system of claim 2, wherein the drive component comprises an electromechanical system.

5. The multiple stage drug delivery system of claim 1, wherein the cannula (112) couples the second liquid drug stored in the second chamber (116) for delivery when the cannula (112) accesses the second chamber (116).

6. The multiple stage drug delivery system of any one of claims 1 to 5, wherein the first plunger (104) is positioned adjacent to the second plunger (106) after the first drug (108) is extracted from the first chamber (114).

7. The multiple stage drug delivery system of any one of claims 1 to 6, wherein the drive component is configured to advance the first plunger (104) and the second plunger (106) with the cannula (112) toward an end of the cartridge (102), thereby extracting the second liquid drug (110) from the second chamber (116) through the cannula (112).

8. The multiple stage drug delivery system of any one of claims 1 to 7, wherein the first drug (108) is dispensed over a first amount of time at a first rate and the second drug (110) is dispensed over a second amount of time at a second rate.

9. The multiple stage drug delivery system of claim 8, wherein the first amount of time and the second amount of time are non-overlapping.

10. The multiple stage drug delivery system of claim 8 or 9, wherein the first and second rates are different.

11. The multiple stage drug delivery system of any preceding claim, wherein the first (104) and second plungers (106) are elastomeric plungers.

12. The multiple stage drug delivery system of any preceding claim, wherein the first (108) and second (110) liquid drugs are different drugs.

13. The multiple stage drug delivery system of any preceding claim, wherein the first chamber (114) and the second chamber (116) have different volumes.

14. The multiple stage drug delivery system of any preceding claim, wherein the cartridge (102) comprises glass.

## Patentansprüche

1. Mehrstufiges Arzneimittelverabreichungssystem, das Folgendes umfasst:
eine Patrone (102);
einen ersten Kolben (104), der in der Patrone (102) positioniert ist;
einen zweiten Kolben (106), der in der Patrone (102) positioniert ist, wobei der erste Kolben (104), der zweite Kolben (106) und die Patrone (102) eine erste Kammer (114) bilden, die zum Speichern eines ersten flüssigen Arzneimittels (108) ausgelegt ist, und der zweite Kolben (106) und die Patrone (102) eine zweite Kammer (116) bilden, die zum Speichern eines zweiten flüssigen Arzneimittels (110) ausgelegt ist;
eine Kanüle (112), die dazu ausgelegt ist, den ersten Kolben (104) zu durchstoßen, um durch Anwenden einer Kraft (202) auf die bewegliche Kanüle (112) auf das in der ersten Kammer gespeicherte erste flüssige Arzneimittel (108) zuzugreifen; und
eine Kanüleneinführungskomponente, die mit der Kanüle (112) gekoppelt ist und eine oder mehrere Federn beinhaltet, wobei die Kanüleneinführungskomponente dazu ausgelegt ist, als Reaktion darauf, dass sie ausgelöst wird, zu bewirken, dass die Kanüle (112) den ersten Kolben (104) durchsticht, wobei:
der erste Kolben (104) zu Folgendem ausgelegt ist:
sich in Richtung des zweiten Kolbens (106) zu bewegen, und
das erste flüssige Arzneimittel (108) aus der ersten Kammer (114) durch die Kanüle (112) in eine Richtung auszustoßen, die im Wesentlichen entgegengesetzt zu der Richtung der Bewegung des ersten Kolbens (104) zu dem zweiten Kolben (106) ist, und
wobei die Kanüle (112) ferner ausgelegt ist, als Reaktion auf eine zweite Kraft (502), die auf die Kanüle (112) ausgeübt wird, den zweiten Kolben (106) zu durchstechen, um auf das zweite flüssige Arzneimittel (110), das in der zweiten Kammer (116) gespeichert ist, zuzugreifen, nachdem das erste flüssige Arzneimittel (108) aus der ersten Kammer (114) extrahiert wurde.

2. Mehrstufiges Arzneimittelverabreichungssystem nach Anspruch 1, das ferner eine Antriebskomponente umfasst, die dazu ausgelegt ist, den ersten Kolben (104) mit der Kanüle in Richtung des zweiten Kolbens (106) vorzuschieben, wodurch das erste flüssige Arzneimittel (108) aus der ersten Kammer (114) durch die Kanüle (112) extrahiert wird.

3. Mehrstufiges Arzneimittelverabreichungssystem nach Anspruch 2, wobei die Antriebskomponente eine oder mehrere Federn umfasst.

4. Mehrstufiges Arzneimittelverabreichungssystem nach Anspruch 2, wobei die Antriebskomponente ein elektromechanisches System umfasst.

5. Mehrstufiges Arzneimittelverabreichungssystem nach Anspruch 1, wobei die Kanüle (112) das in der zweiten Kammer (116) gespeicherte zweite flüssige Arzneimittel zur Verabreichung koppelt, wenn die Kanüle (112) auf die zweite Kammer (116) zugreift.

6. Mehrstufiges Arzneimittelverabreichungssystem nach einem der Ansprüche 1 bis 5, wobei der erste Kolben (104) benachbart zu dem zweiten Kolben (106) positioniert ist, nachdem das erste Arzneimittel (108) aus der ersten Kammer (114) extrahiert wurde.

7. Mehrstufiges Arzneimittelverabreichungssystem nach einem der Ansprüche 1 bis 6, wobei die Antriebskomponente dazu ausgelegt ist, den ersten Kolben (104) und den zweiten Kolben (106) mit der Kanüle (112) in Richtung eines Endes der Patrone (102) vorzuschieben, wodurch das zweite flüssige Arzneimittel (110) aus der zweiten Kammer (116) durch die Kanüle (112) extrahiert wird.

8. Mehrstufiges Arzneimittelverabreichungssystem nach einem der Ansprüche 1 bis 7, wobei das erste Arzneimittel (108) über eine erste Zeitdauer mit einer ersten Rate abgegeben wird und das zweite Arzneimittel (110) über eine zweite Zeitdauer mit einer zweiten Rate abgegeben wird.

9. Mehrstufiges Arzneimittelverabreichungssystem nach Anspruch 8, wobei die erste Zeitdauer und die zweite Zeitdauer nicht überlappen.

10. Mehrstufiges Arzneimittelverabreichungssystem nach Anspruch 8 oder 9, wobei die erste und die zweite Rate verschieden sind.

11. Mehrstufiges Arzneimittelverabreichungssystem nach einem der vorhergehenden Ansprüche, wobei der erste (104) und der zweite Kolben (106) Elastomerkolben sind.

12. Mehrstufiges Arzneimittelverabreichungssystem nach einem der vorhergehenden Ansprüche, wobei das erste (108) und das zweite (110) flüssige Arzneimittel unterschiedliche Arzneimittel sind.

13. Mehrstufiges Arzneimittelverabreichungssystem nach einem der vorhergehenden Ansprüche, wobei die erste Kammer (114) und die zweite Kammer (116) unterschiedliche Volumina aufweisen.

14. Mehrstufiges Arzneimittelverabreichungssystem nach einem der vorhergehenden Ansprüche, wobei die Patrone (102) Glas umfasst.

## Revendications

1. Système de distribution de médicament multi-étapes, comprenant :
une cartouche (102),
un premier piston (104) positionné dans la cartouche (102) ;
un second piston (106) positionné dans la cartouche (102), le premier piston (104), le second piston (106) et la cartouche (102) formant une première chambre (114) configurée pour stocker un premier médicament liquide (108), et le second piston (106) et la cartouche (102) formant une seconde chambre (116) configurée pour stocker un second médicament liquide (110) ;
une canule (112) configurée pour percer à travers le premier piston (104) afin d'accéder au premier médicament liquide (108) stocké dans la première chambre par application d'une force (202) sur la canule mobile (112) ; et
un composant d'insertion de canule couplé à la canule (112) et comprenant un ou plusieurs ressorts, le composant d'insertion de canule étant configuré pour, en réponse à un déclenchement, amener la canule (112) à percer le premier piston (104),
le premier piston (104) étant configuré pour :
se déplacer vers le second piston (106), et
expulser le premier médicament liquide (108) de la première chambre (114) à travers la canule (112) dans une direction sensiblement opposée à la direction du mouvement du premier piston (104) vers le second piston (106), et
la canule (112) étant en outre configurée en réponse à une seconde force (502) appliquée à la canule (112) pour percer le second piston (106) afin d'accéder au second médicament liquide (110) stocké dans la seconde chambre (116) après que le premier médicament liquide (108) a été extrait de la première chambre (114).

2. Système de distribution de médicament multi-étapes selon la revendication 1, comprenant en outre un composant d'entraînement configuré pour faire avancer le premier piston (104) avec la canule vers le second piston (106), extrayant ainsi le premier médicament liquide (108) de la première chambre (114) à travers la canule (112).

3. Système de distribution de médicament multi-étapes selon la revendication 2, le composant d'entraînement comprenant un ou plusieurs ressorts.

4. Système de distribution de médicament multi-étapes selon la revendication 2, le composant d'entraînement comprenant un système électromécanique.

5. Système de distribution de médicament multi-étapes selon la revendication 1, la canule (112) couplant le second médicament liquide stocké dans la seconde chambre (116) pour la distribution lorsque la canule (112) accède à la seconde chambre (116).

6. Système de distribution de médicament multi-étapes selon l'une quelconque des revendications 1 à 5, le premier piston (104) étant positionné de manière adjacente au second piston (106) après que le premier médicament (108) a été extrait de la première chambre (114).

7. Système de distribution de médicament multi-étapes selon l'une quelconque des revendications 1 à 6, le composant d'entraînement étant configuré pour faire avancer le premier piston (104) et le second piston (106) avec la canule (112) vers une extrémité de la cartouche (102), extrayant ainsi le second médicament liquide (110) de la seconde chambre (116) à travers la canule (112).

8. Système de distribution de médicament multi-étapes selon l'une quelconque des revendications 1 à 7, le premier médicament (108) étant distribué au cours d'une première quantité de temps à un premier débit et le second médicament (110) étant distribué au cours d'une seconde quantité de temps à un second débit.

9. Système de distribution de médicament multi-étapes selon la revendication 8, la première quantité de temps et la seconde quantité de temps ne se chevauchant pas.

10. Système de distribution de médicament multi-étapes selon la revendication 8 ou 9, les premier et second débit étant différents.

11. Système de distribution de médicament multi-étapes selon l'une quelconque des revendications précédentes, les premier (104) et second pistons (106) étant des pistons élastomères.

12. Système de distribution de médicament multi-étapes selon l'une quelconque des revendications précédentes, les premier (108) et second (110) médicaments liquides étant des médicaments différents.

13. Système de distribution de médicament multi-étapes selon l'une quelconque des revendications précédentes, la première chambre (114) et la seconde chambre (116) ayant des volumes différents.

14. Système de distribution de médicament multi-étapes selon l'une quelconque des revendications précédentes, la cartouche (102) comprenant du verre.
